# EUROPÄISCHE PATENTSCHRIFT

(11) **EP 2 613 133 B1**
(45) Veröffentlichungstag und Bekanntmachung des Hinweises auf die Patenterteilung: **18.03.2015**
(21) Anmeldenummer: 12150079.7
(22) Anmeldetag: 03.01.2012
(51) Int. Cl.: G01N 1/28, G01N 3/02

(54) **Herstellung von Vergleichstestkörpern zur zerstörungsfreien Prüfung mit repräsentativen Rissen bezüglich ihrer Orientierung und Untersuchungsverfahren**
Production of comparative test bodies for non-destructive testing with representative fissures regarding their orientation and test method
Fabrication de corps de test comparatif pour le contrôle non destructif avec des fissures représentatives relatives à leur orientation et procédé d'examen

(43) Veröffentlichungstag der Anmeldung: 10.07.2013
(73) Patentinhaber: Siemens Aktiengesellschaft, 80333 München (DE)
(72) Erfinder: Böhme, Christian, 46119 Oberhausen (DE); Clossen-von Lanken Schulz, Michael, 47661 Issum (DE); Jakielski, Sebastian, 46238 Bottrop (DE); Obermayr, Stefan, 45475 Mülheim (DE)

(56) Entgegenhaltungen:
- EP-A2- 0 631 123
- JP-A- 2007 155 540
- SU-A1- 561 109
- SU-A1- 1 227 974

## Beschreibung

Die Erfindung betrifft die Herstellung eines Vergleichstestkörpers, der im Rahmen einer zerstörungsfreien Prüfung als Vergleichsprobe genutzt werden kann und ein Untersuchungsverfahren.

Die SU 561109 offenbart eine Platte mit einem Schlitz, wobei am Ende des Schlitzes ein Riss wachsen gelassen wird.

Die SU 1227974 A1 offenbart eine Platte mit einem Schlitz mit einer kreisförmigen Öffnung, an deren Ende einen Riss wachsen gelassen wird.

In der zerstörungsfreien Untersuchung, insbesondere mittels Ultraschall, sollen Risse detektiert werden, wobei die Risslänge und die Rissorientierung wichtige Informationen darstellen. Auf realen Bauteilen wie Turbinenschaufeln sind die Oberflächen fast immer gekrümmt, was bezüglich der Messerfassung wiederum eine Einflussgröße darstellt.

Es ist daher Aufgabe der Erfindung einen Vergleichstestkörper aufzuzeigen, mit dem eine kontrollierte Risslänge bzgl. Orientierung an gekrümmten Oberflächen hergestellt werden kann und Untersuchungsverfahren verbessert werden können.

Die Aufgabe wird gelöst durch ein Verfahren gemäß Anspruch 1 und Untersuchungsverfahren gemäß Anspruch 3.

In den Unteransprüchen sind weitere vorteilhafte Maßnahmen aufgelistet, die beliebig miteinander kombiniert werden können, um weitere Vorteile zu erzielen.

Es zeigen:
- Figur 1 - 5: Ausführungsbeispiele bzw. Erläuterungen der Erfindung,
- Figur 6: eine Turbinenschaufel,
- Figur 7: eine Liste von Superlegierungen.

Die Figuren und die Beschreibung stellen nur Ausführungsbeispiele der Erfindung dar.

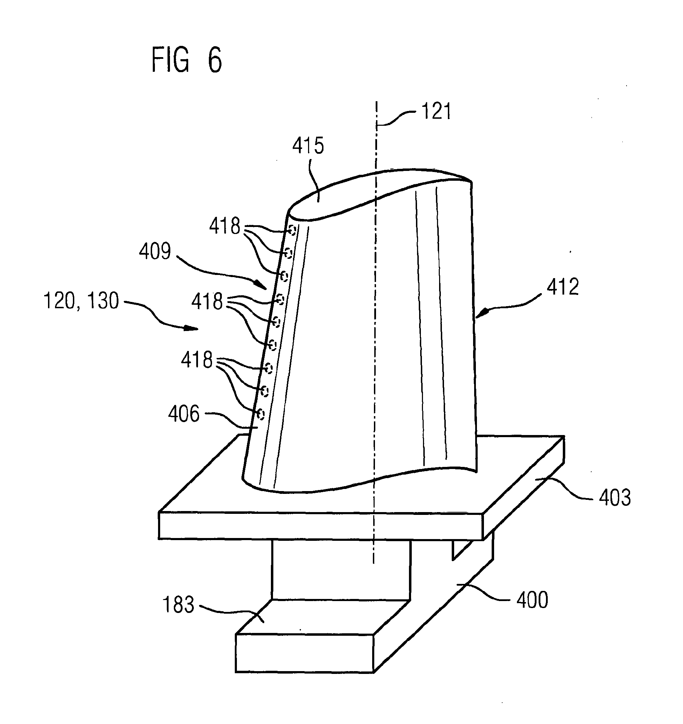

Turbinenschaufeln 120, 130 (Fig. 6) können nach starker Beanspruchung trotz Schutzschichten einen Riss aufweisen. Zur Entscheidung, ob die Turbinenschaufel 120, 130 repariert werden kann und wie sie repariert werden muss, ist es sinnvoll, vorab Risse zu detektieren und zu bestimmen. Eine besonders kritische Stelle ist die sogenannte "Fillet Region", der Übergang zwischen Plattform 403 und Schaufelblatt 406 (Fig. 6) oder allgemein gekrümmte Flächen.

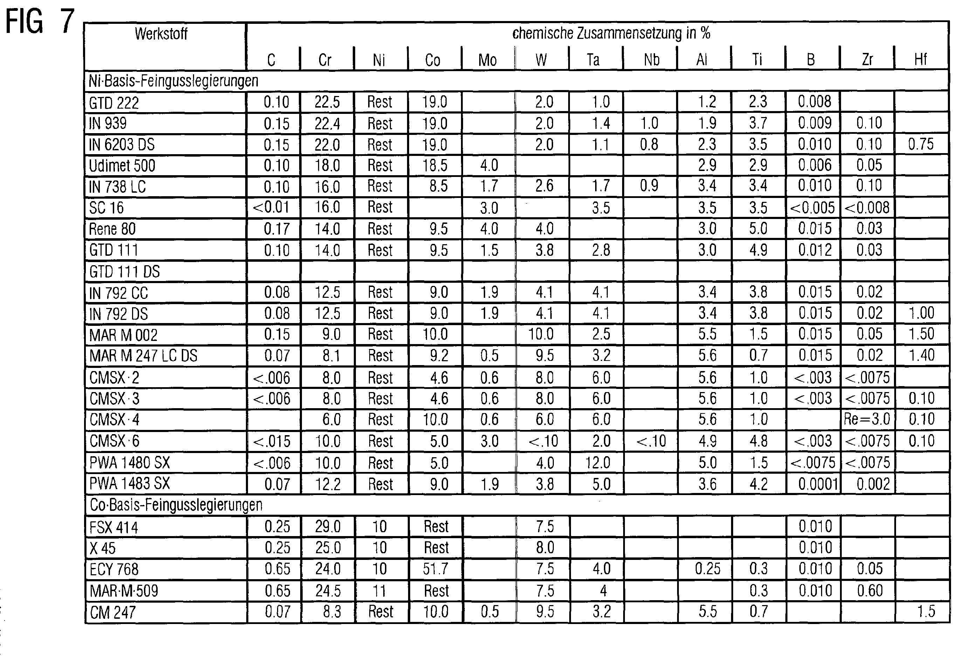

Turbinenschaufeln weisen kobalt- oder nickelbasierte Superlegierungen auf, insbesondere gemäß Figur 7.

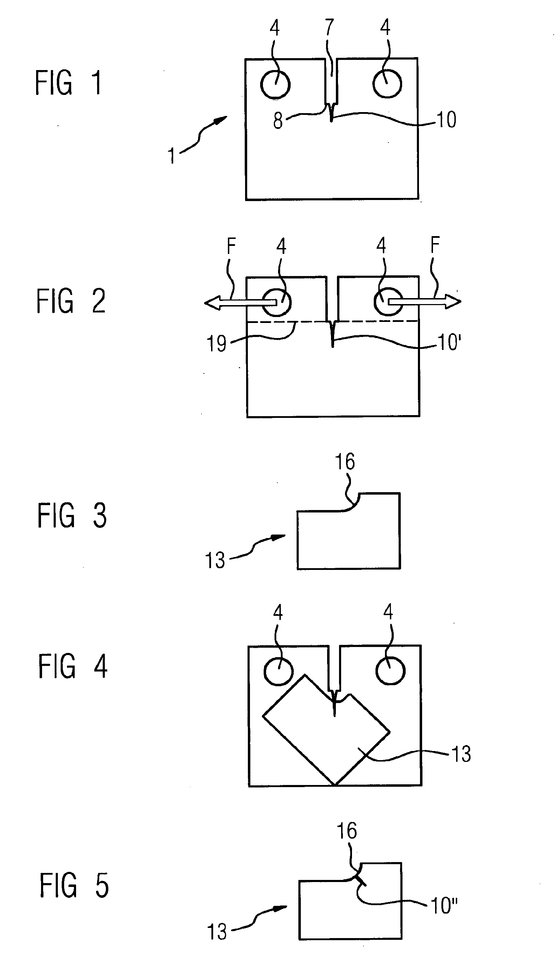

Figur 3 zeigt einen gewünschten Vergleichstestkörper 13 mit einem Bereich 16, der die "Fillet Region" einer Turbinenschaufel 120, 130 darstellt.
In diesen Bereich 16 soll vorzugsweise ein Riss 10" (Fig. 5) eingebracht werden, so dass der Vergleichstestkörper 13 mit einem Riss 10" als Vergleich zur Verfügung steht.

In Figur 1 und 2 ist gezeigt, wie standardmäßig in eine Platte 1 (vorzugsweise rechteckig) Risse 10, 10' kontrolliert eingebracht werden.
Dabei wird in eine metallische Platte 1, insbesondere aus einer nickelbasierten Legierung, ein Schlitz 7 mechanisch eingebracht und an Haltepunkten 4 wird eine Kraft angelegt, die den Schlitz 7 aufweitet, so dass ein Riss 10, 10' wächst. Die Risslänge kann aufgrund von Erfahrungswerten kontrolliert bezüglich Orientierung (in der Regel in Rissrichtung zum Schlitz 7) und Länge eingebracht werden.

Jedoch würde ein solcher Vergleichstestkörper gemäß Figur 2, wenn er an der gestrichelten Linie 19 abgeschnitten werden würde, also an der Oberfläche, von der der Riss 10' ausgeht, nicht repräsentativ für reale Bauteile sein.

Daher ist die Platte 1 so gewählt, dass eine gewünschte Sollform des Vergleichstestkörpers 13 die gewünschte Lage eines Risses 10" in dem Vergleichstestkörper 13 umfasst, wie es in Figur 4 dargestellt ist.

Die Lage des Vergleichstestkörpers 13 kann immer wieder variiert werden, so dass der Riss 10" an einer anderen Stelle vorhanden ist, eine andere Risslänge oder eine andere Orientierung aufweist. Dementsprechend wird die Größe der Platte 1 und die Länge des Risses 10' gewählt.

Mit einer solchen Platte 1 wird ein Riss 10, 10' erzeugt und dann wird gemäß Figur 5 die Sollform des Vergleichstestkörpers 13 aus der Platte 1 herausgeschnitten, die dann in der "Fillet Region" 16 oder zu Beginn der "Fillet Region" 16 einen definierten und bekannten Riss 10" aufweist.

Eine solche Probe gemäß Figur 5 wird dann zu Vergleichszwecken weitere Ultraschalluntersuchungen oder Wirbelstrommessungen von realen Bauteilen 120, 130 herangezogen.
Dabei werden dann die Signale des realen Bauteils und die des Vergleichstestkörpers 13 (Fig. 5) verglichen.

Gegebenenfalls werden mehrere Vergleichstestkörper 13 hergestellt, die sich in der Orientierung des Risses und/oder Lage des Risses unterscheiden, so dass das reale Messergebnis einem Vergleichsergebnis zugeordnet werden kann.

Die Figur 6 zeigt in perspektivischer Ansicht eine Laufschaufel 120 oder Leitschaufel 130 einer Strömungsmaschine, die sich entlang einer Längsachse 121 erstreckt.

Die Strömungsmaschine kann eine Gasturbine eines Flugzeugs oder eines Kraftwerks zur Elektrizitätserzeugung, eine Dampfturbine oder ein Kompressor sein.

Die Schaufel 120, 130 weist entlang der Längsachse 121 aufeinander folgend einen Befestigungsbereich 400, eine daran angrenzende Schaufelplattform 403 sowie ein Schaufelblatt 406 und eine Schaufelspitze 415 auf.
Als Leitschaufel 130 kann die Schaufel 130 an ihrer Schaufelspitze 415 eine weitere Plattform aufweisen (nicht dargestellt) .

Im Befestigungsbereich 400 ist ein Schaufelfuß 183 gebildet, der zur Befestigung der Laufschaufeln 120, 130 an einer Welle oder einer Scheibe dient (nicht dargestellt).
Der Schaufelfuß 183 ist beispielsweise als Hammerkopf ausgestaltet. Andere Ausgestaltungen als Tannenbaum- oder Schwalbenschwanzfuß sind möglich.
Die Schaufel 120, 130 weist für ein Medium, das an dem Schaufelblatt 406 vorbeiströmt, eine Anströmkante 409 und eine Abströmkante 412 auf.

Bei herkömmlichen Schaufeln 120, 130 werden in allen Bereichen 400, 403, 406 der Schaufel 120, 130 beispielsweise massive metallische Werkstoffe, insbesondere Superlegierungen verwendet.
Solche Superlegierungen sind beispielsweise aus der EP 1 204 776 B1, EP 1 306 454, EP 1 319 729 A1, WO 99/67435 oder WO 00/44949 bekannt.
Die Schaufel 120, 130 kann hierbei durch ein Gussverfahren, auch mittels gerichteter Erstarrung, durch ein Schmiedeverfahren, durch ein Fräsverfahren oder Kombinationen daraus gefertigt sein.

Werkstücke mit einkristalliner Struktur oder Strukturen werden als Bauteile für Maschinen eingesetzt, die im Betrieb hohen mechanischen, thermischen und/oder chemischen Belastungen ausgesetzt sind.
Die Fertigung von derartigen einkristallinen Werkstücken erfolgt z.B. durch gerichtetes Erstarren aus der Schmelze. Es handelt sich dabei um Gießverfahren, bei denen die flüssige metallische Legierung zur einkristallinen Struktur, d.h. zum einkristallinen Werkstück, oder gerichtet erstarrt.
Dabei werden dendritische Kristalle entlang dem Wärmefluss ausgerichtet und bilden entweder eine stängelkristalline Kornstruktur (kolumnar, d.h. Körner, die über die ganze Länge des Werkstückes verlaufen und hier, dem allgemeinen Sprachgebrauch nach, als gerichtet erstarrt bezeichnet werden) oder eine einkristalline Struktur, d.h. das ganze Werkstück besteht aus einem einzigen Kristall. In diesen Verfahren muss man den Übergang zur globulitischen (polykristallinen) Erstarrung meiden, da sich durch ungerichtetes Wachstum notwendigerweise transversale und longitudinale Korngrenzen ausbilden, welche die guten Eigenschaften des gerichtet erstarrten oder einkristallinen Bauteiles zunichte machen.
Ist allgemein von gerichtet erstarrten Gefügen die Rede, so sind damit sowohl Einkristalle gemeint, die keine Korngrenzen oder höchstens Kleinwinkelkorngrenzen aufweisen, als auch Stängelkristallstrukturen, die wohl in longitudinaler Richtung verlaufende Korngrenzen, aber keine transversalen Korngrenzen aufweisen. Bei diesen zweitgenannten kristallinen Strukturen spricht man auch von gerichtet erstarrten Gefügen (directionally solidified structures).
Solche Verfahren sind aus der US-PS 6,024,792 und der EP 0 892 090 A1 bekannt.

Ebenso können die Schaufeln 120, 130 Beschichtungen gegen Korrosion oder Oxidation aufweisen, z. B. (MCrAlX; M ist zumindest ein Element der Gruppe Eisen (Fe), Kobalt (Co), Nickel (Ni), X ist ein Aktivelement und steht für Yttrium (Y) und/oder Silizium und/oder zumindest ein Element der Seltenen Erden, bzw. Hafnium (Hf)). Solche Legierungen sind bekannt aus der EP 0 486 489 B1, EP 0 786 017 B1, EP 0 412 397 B1 oder EP 1 306 454 A1.
Die Dichte liegt vorzugsweise bei 95% der theoretischen Dichte.
Auf der MCrAlX-Schicht (als Zwischenschicht oder als äußerste Schicht) bildet sich eine schützende Aluminiumoxidschicht (TGO = thermal grown oxide layer).
Vorzugsweise weist die Schichtzusammensetzung Co-30Ni-28Cr-8Al-0,6Y-0,7Si oder Co-28Ni-24Cr-10Al-0,6Y auf. Neben diesen kobaltbasierten Schutzbeschichtungen werden auch vorzugsweise nickelbasierte Schutzschichten verwendet wie Ni-10Cr-12Al-0,6Y-3Re oder Ni-12Co-21Cr-11Al-0,4Y-2Re oder Ni-25Co-17Cr-10Al-0,4Y-1,5Re.

Auf der MCrAlX kann noch eine Wärmedämmschicht vorhanden sein, die vorzugsweise die äußerste Schicht ist, und besteht beispielsweise aus ZrO₂, Y₂O₃-ZrO₂, d.h. sie ist nicht, teilweise oder vollständig stabilisiert durch Yttriumoxid und/oder Kalziumoxid und/oder Magnesiumoxid.
Die Wärmedämmschicht bedeckt die gesamte MCrAlX-Schicht. Durch geeignete Beschichtungsverfahren wie z.B. Elektronenstrahlverdampfen (EB-PVD) werden stängelförmige Körner in der Wärmedämmschicht erzeugt.
Andere Beschichtungsverfahren sind denkbar, z.B. atmosphärisches Plasmaspritzen (APS), LPPS, VPS oder CVD. Die Wärmedämmschicht kann poröse, mikro- oder makrorissbehaftete Körner zur besseren Thermoschockbeständigkeit aufweisen. Die Wärmedämmschicht ist also vorzugsweise poröser als die MCrAlX-Schicht.

Wiederaufarbeitung (Refurbishment) bedeutet, dass Bauteile 120, 130 nach ihrem Einsatz gegebenenfalls von Schutzschichten befreit werden müssen (z.B. durch Sandstrahlen). Danach erfolgt eine Entfernung der Korrosions- und/oder Oxidationsschichten bzw. -produkte. Gegebenenfalls werden auch noch Risse im Bauteil 120, 130 repariert. Danach erfolgt eine Wiederbeschichtung des Bauteils 120, 130 und ein erneuter Einsatz des Bauteils 120, 130.

Die Schaufel 120, 130 kann hohl oder massiv ausgeführt sein. Wenn die Schaufel 120, 130 gekühlt werden soll, ist sie hohl und weist ggf. noch Filmkühllöcher 418 (gestrichelt angedeutet) auf.

## Patentansprüche

1. Verfahren zur Herstellung eines Vergleichstestkörpers (13) für eine zerstörungsfreie Prüfung,
bei dem in eine Platte (1) ein Schlitz (7) eingebracht und durch Krafteinwirkung ein Riss (10, 10') am Ende (8) des Schlitzes (7) in der Platte (1) hinein wachsen gelassen wird,
wobei die Platte (1) groß genug ist,
dass eine Sollform des Vergleichstestkörpers (13) aus der Platte (1) in der gewünschten Orientierung bezüglich des Risses (10') nach Einbringung des Risses (10') herausgearbeitet werden kann, **dadurch gekennzeichnet, dass** dann der Vergleichstestkörper (13) so aus der Platte (1) herausgeschnitten wird,
dass ein Riss (10'') in der gewünschten Länge und/oder Lage,
insbesondere in einem gekrümmten Flächenbereich (16),
im Vergleichstestkörper (13) vorhanden ist.

2. Verfahren nach Anspruch 1,
bei dem die Platte (1) rechteckig ist.

3. Verfahren zur Untersuchung von Bauteilen mit gekrümmten Flächen (16),
mit den Schritten
a) Herstellung eines Vergleichskörpers nach Anspruch 1 oder 2,
b) Vergleichen des Vergleichskörpers mit einem realen Bauteil,
das einen Riss aufweist,
wobei die Signale von Ultraschalluntersuchungen oder Wirbelstrommessungen miteinander verglichen werden,
so dass das reale Messergebnis einen Vergleichsergebnis zugeordnet werden kann.

4. Verfahren nach Anspruch 3,
bei dem mehrere Vergleichstestkörper (13) mit unterschiedlichen Rissen als Vergleich herangezogen werden.

## Claims

1. Method for producing a comparative test body (13) for nondestructive testing,
in which a slot (7) is introduced into a plate (1) and, by means of the action of force, a fissure (10, 10') at the end (8) of the slot (7) in the plate (1) is allowed to grow inwards,
the plate (1) being sufficiently large
that an intended shape of the comparative test body (13) can be machined out of the plate (1) in the desired orientation with respect to the fissure (10') following the introduction of the fissure (10'), **characterized in that** the comparative test body (13) is then cut out of the plate (1) in such a way
that a fissure (10'') of the desired length and/or position
is present in the comparative test body (13),
in particular in a curved surface area (16).

2. Method according to Claim 1,
in which the plate (1) is rectangular.

3. Method for testing components having curved surfaces (16), comprising the steps
a) producing a comparative body according to Claim 1 or 2,
b) comparing the comparative body with a real component which has a fissure,
the signals from ultrasonic tests or eddy current measurements being compared with one another,
so that the real measured result can be assigned to a comparative result.

4. Method according to Claim 3,
in which a plurality of comparative test bodies (13) having different fissures are used as a comparison.

## Revendications

1. Procédé de fabrication d'un corps (13) de test comparatif pour un contrôle sans destruction,
dans lequel on ménage, dans un plateau ( 1 ), une fente ( 7 ) et, par action d'une force, on fait croître une fissure ( 10, 10' ) à l'extrémité ( 8 ) de la fente ( 7 ) dans le plateau ( 1 ),
dans lequel le plateau ( 1 ) est suffisamment grand,
pour pouvoir usiner une forme de consigne du corps ( 13 ) de test comparatif dans le plateau ( 1 ) en l'orientation souhaitée par rapport à la fissure ( 10' ), après avoir ménagé la fissure ( 10' ),
**caractérisé en ce qu'**ensuite on découpe le corps ( 13 ) de test comparatif dans le plateau ( 1 ),
de manière à ce qu'il y ait une fissure ( 10'' ) de la longueur et/ou de la position souhaitée,
notamment dans une zone ( 16 ) de surface courbée dans le corps ( 13 ) de test comparatif.

2. Procédé suivant la revendication 1,
dans lequel le plateau ( 1 ) est rectangulaire.

3. Procédé d'examen de pièce ayant des surfaces ( 16 ) courbées,
comprenant les stades
a) fabrication d'un corps comparatif suivant la revendication 1 ou 2,
b) comparaison du corps comparatif à une pièce réelle,
qui a une fissure,
dans lequel on compare entre eux les signaux d'examen par ultrasons ou des mesures de courant de Foucault,
de manière à ce que le résultat de mesure réelle puisse être affecté à un résultat de comparaison.

4. Procédé suivant la revendication 3,
dans lequel on tire profit, comme comparaison, de plusieurs corps ( 13 ) de test comparatif ayant des fissures différentes.
